# EUROPEAN PATENT SPECIFICATION

(11) **EP 4 316 359 B1**
(45) Date of publication and mention of the grant of the patent: **02.09.2026**
(21) Application number: 22929010.1
(22) Date of filing: 28.02.2022
(51) Int. Cl.: A61B 5/103, A61B 5/11, A61B 5/00

(54) **SYSTEM FOR MEASURING POSTURE/BALANCE BY USING SUBJECT SAFETY DEVICE**
SYSTEM ZUR MESSUNG VON HALTUNG/BALANCE UNTER VERWENDUNG EINER PERSONENSICHERHEITSVORRICHTUNG
SYSTÈME DE MESURE DE POSTURE/D'ÉQUILIBRE À L'AIDE D'UN DISPOSITIF DE SÉCURITÉ DE SUJET

(43) Date of publication of application: 07.02.2024
(73) Proprietor: Osong Medical Innovation Foundation, Cheongju-si, Chungcheongbuk-do 28160 (KR)
(72) Inventor: KIM, Young Jin, Cheongju-si Chungcheongbuk-do 28160 (KR); KIM, Seung Pyo, Gwangju 61260 (KR); MOON, Dong Jun, Sejong 30098 (KR); JUNG, Ha Chul, Cheongju-si Chungcheongbuk-do 28427 (KR); AHN, Jin Woo, Cheongju-si Chungcheongbuk-do 28160 (KR); KANG, Shin Il, Cheongju-si Chungcheongbuk-do 28160 (KR)
(74) Representative: Lewis Silkin LLP
(86) International application number: PCT/KR2022/002888
(87) International publication number: WO 2023/163258

(56) References cited:
- KR-A- 20120 081 702
- KR-A- 20190 081 350
- KR-A- 20200 084 101
- KR-B1- 101 476 889
- KR-B1- 102 270 146
- US-A1- 2021 005 106
- US-B2- 10 272 284
- US-B2- 10 376 222
- US-B2- 10 470 667

## Description

### BACKGROUND

### 1. Field of Disclosure

The present disclosure relates to a posture balance measurement system using a safe device for a subject and a posture balance measurement method using the system, and more specifically the present disclosure relates to a posture balance measurement system using a safe device for a subject and a posture balance measurement method using the system configured to measure balancing function of the subject using a harness at which the subject is fixed and a footrest on which the subject is located, for testing the subject's balancing function such as dizziness.

### 2. Description of Related Technology

Generally, the sensory organization test uses the center of pressure of the footrest to selectively apply stimuli under a total of 6 conditions for visual, somatic, and tactile sensations, and judges the result based on the equilibrium score in each condition.

The sensory organization test is a representative test method for examining equilibrium functions such as dizziness. For example, as disclosed in Korean Patent No. 10-0921512, in the sensory organization test, a number of test methods are applied in which the test is performed by measuring only the plantar pressure using a plantar pressure measuring device.

However, in the case of testing such equilibrium function only with the plantar pressure of the footrest where the subject is located, the subject may be separated from the footrest during the examination process, or may lean on a support such as a safety device or a wall located near the examination device, if the subject is elderly or has severe symptoms of dizziness or psychological instability. Thus, when this situation occurs, an error in the measured equilibrium score may occur, and a problem of an increase in inspection time may be caused.

Accordingly, there is a problem in that the balance function test result is not accurate by simply measuring plantar pressure while the subject is positioned on the footrest, and repeated measurements are required, causing inconvenience and inefficiency to both the subject and the examiner.

Related prior art is Korean Patent No. 10-0921513.

US20210005106A1 discloses a motion support system that includes a walking assistance apparatus as an assistance apparatus. The assistance apparatus assists a motion of a joint performed by a user. The assistance level setting unit sets an assistance level that is a level of a force exerted by the assistance apparatus. The load data acquisition unit acquires load data related to a load of the assistance apparatus. The displacement sensor detects a displacement of the joint. The control unit determines a recommended assistance level, which is an assistance level to be recommended, based on the assistance level, the load data, and an output of the displacement sensor, and outputs information about the determined recommended assistance level.

KR1020200084101A discloses an equilibrium inspection system for performing human body equilibrium inspection.

KR1020120081702A discloses apparatus of measuring body of postural balance.

KR1020190081350A discloses a system for monitoring a walking balance for lower extremity rehabilitation.

KR101476889B1 discloses a walking assistance device and a drive method thereof.

KR102270146B1 discloses a posture measuring system for measuring a balance function of a subject based on a planar pressure and a wire rod and a posture measuring method using the same. Further relevant prior art is disclosed in US 10 376 222 B2, US 10 470 667 B2, US 10 272 284 B2.

### SUMMARY

The present invention is developed to solve the above-mentioned problems of the related arts. The present invention is defined in claim 1 and provides a posture balance measurement system using a safe device for a subject, capable of measure balancing function of the subject using a harness at which the subject is fixed and a footrest on which the subject is located, for testing the subject's balancing function such as dizziness, so that a more accurate examination may be performed and the subject's body may be fixed and measured at the same time without installing a separate measurement module.

In addition, the present disclosure provides a posture balance measurement method using the system.

According to an example embodiment, the posture balance measurement system includes a plate, a pressure measurement unit, a harness, a balance measurement unit, a controller and an evaluation part. The plate is configured to support a subject in an upright standing posture. The pressure measurement unit is disposed on the plate, and is configured to measure a plantar pressure of the subject. The harness is configured to fix and support a body of the subject. The balance measurement unit is disposed at the harness, and is configured to measure balance information of the subject. The controller is configured to control the pressure measurement unit and the balance measurement unit in accordance with the performance of a test. The evaluation part is configured to determine a posture balance state of the subject, based on pressure information of the subject obtained by the pressure measurement unit, and the balance information of the subject obtained by the balance measurement unit. The balance measurement unit comprises a balance information measurement part, and the balance information measurement part is configured to measure a balance state of the subject in which the subject's standing posture or balance posture changes according to the test performance when the subject is fixed to the harness. The balance information measurement part comprises a gyroscope and at least one of an accelerometer and a magnetometer mounted on the harness, for measuring the balance state of the subject. The balance information measurement part further comprises a first joint sensor disposed at a femoral region of the subject, a second joint sensor disposed at a shin of the subject, and a third joint sensor disposed at a foot of the subject. The first joint sensor, together with the gyroscope, is configured to measure a joint angle between a waist and the femoral region of the subject. The first joint sensor is further configured to, together with the second joint sensor, measure a joint angle between the femoral region and the shin of the subject. The second joint sensor is further configured to, together with the third joint sensor, measure a joint angle between the shin and the foot of the subject.

In an example, the pressure measurement unit may include a posture control part configured to adjust a posture of the plate to fit a posture of the subject and to initialize the pressure measurement unit, when the subject stands upright on the plate, a pressure measurement part configured to the plantar pressure of the subject according to the test performance, a pressure change analysis part configured to analyze a pressure change based on the measured plantar pressure, and a pressure information storage part configured to store the measured pressure and the pressure change.

In an example, the balance measurement unit may include a balance information initialization part configured to initialize the balance measurement unit to fit a posture of the subject, when the subject stands upright on the plate, a balance change analysis part configured to analyze a balance change based on the measured balance state, and a balance information storage part configured to store the measured balance state and the balance change.

In an example, the harness may include a pair of first and second fixing parts configured to fix an upper portion of the subject, and a third fixing part connected to the first and second fixing parts, and configured to fix an abdomen portion of the subject.

In an example, the balance measurement unit may be disposed at the third fixing part.

According to the present example embodiments, conventional harnesses only serve to support the subject and plantar pressure is measured using only the lower plate, and thus when the equilibrium function is tested simply using only plantar pressure, the accuracy of the test may be decreased. Thus, the present example embodiments may solve the above problem.

In other words, by providing a balance measurement unit on a harness supporting the subject, it is possible to measure balance information as well as fixing the upper part of the subject, so that the postural balance of the subject may be more accurately measured together with the plantar pressure information.

### BRIEF DESCRIPTION OF THE DRAWINGS

FIG. 1 is a block diagram illustrating a posture balance measurement system according to an example embodiment of the present invention;
FIG. 2 is a perspective view illustrating an example of the posture balance measurement system of FIG. 1;
FIG. 3 is a schematic view illustrating an example state of the subject fixed to a harness and a hardware of a balance information measurement unit of FIG. 2;
FIG. 4 is a schematic view illustrating another example state of the subject fixed to a harness and a hardware of a balance information measurement unit of FIG. 2;
FIG. 5A, FIG. 5B and FIG. 5C are graphs showing example results of the posture balance measurement measured by the balance information measurement unit of FIG. 4;
FIG. 6 is a flow chart showing a posture balance measurement method using the system of FIG. 1; and
FIG. 7 shows images of 6 conditions used in the sensory organization test.

### * Reference numerals

| | | | |
|---|---|---|---|
| 10 : | posture balance measurement system | 20 : | wire |
| 40 : | frame | 50 : | plate |
| 60 : | harness | 70 : | information input part |
| 100 : | pressure measurement unit | 200 | : balance measurement unit |
| 300 : | controller | 400 | : evaluation part |

### DETAILED DESCRIPTION

The invention is described more fully hereinafter with Reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art. In the drawings, the size and relative sizes of layers and regions may be exaggerated for clarity.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section.

The terminology used herein is for the purpose of describing particular embodiments only and is not intended to be limiting of the invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, the invention is described more fully hereinafter with reference to the accompanying drawings, in which embodiments of the invention are shown.

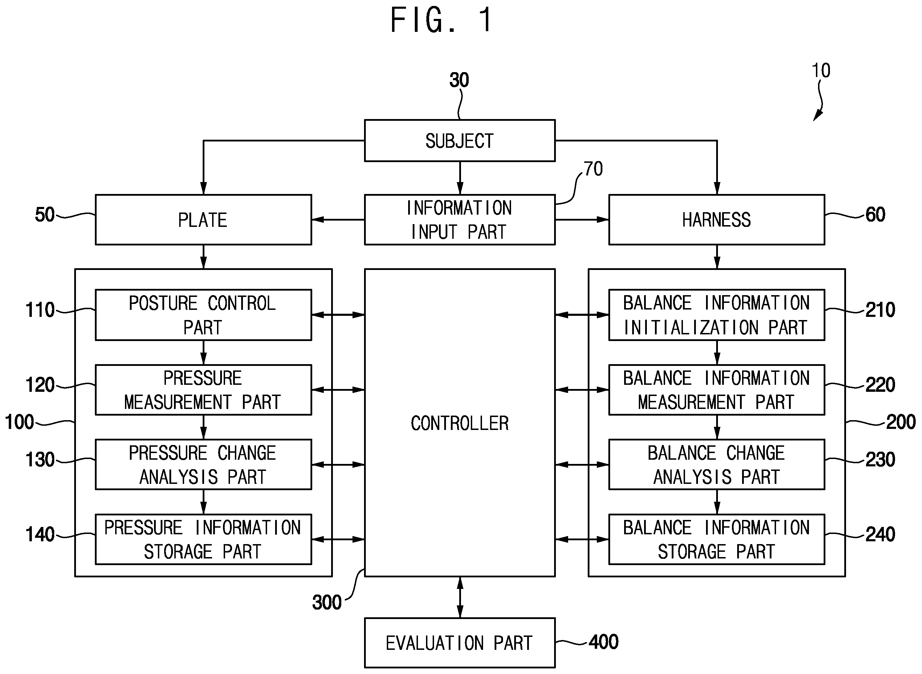

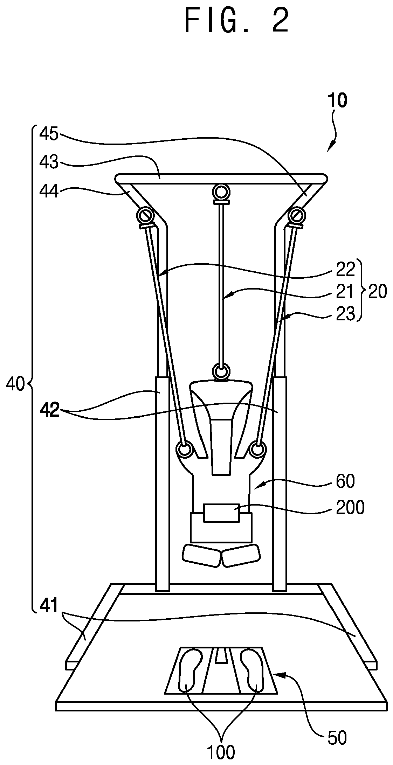

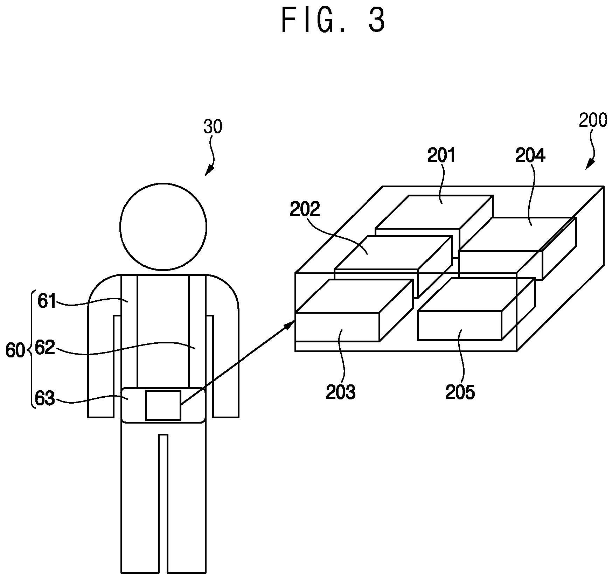

FIG. 1 is a block diagram illustrating a posture balance measurement system according to an example embodiment of the present invention. FIG. 2 is a perspective view illustrating an example of the posture balance measurement system of FIG. 1. FIG. 3 is a schematic view illustrating an example state of the subject fixed to a harness and a hardware of a balance information measurement unit of FIG. 2.

Referring to FIG. 1 and FIG. 2, the posture balance measurement system 10 (hereinafter, the system) includes a wire 20, a frame 40, a plate 50, a harness 60, an information input part 70, a pressure measurement unit 100, a balance measurement unit 200, a controller 300 and an evaluation part 400.

The frame 40 forms a space in which a subject 30 is located, and is a structure at which the harness 60 and the wire 20 are fixed. As illustrated in FIG. 2, the frame 40 includes a lower frame 41, a side frame 42 and upper frames 43, 44 and 45.

The lower frame 41 is located on a ground, and entirely fixes and supports the frame 40. The side frame 42 extends from the lower frame 41 upwardly, and forms a side of the frame 40.

The upper frames 43, 44 and 45 are connected to an upper portion of the side frame 42, and the wire 20 is directly fixed at the upper frames.

The wire 20 includes three wires of first, second and third wires 21, 22 and 23, and the first to third wires 21, 22 and 23 are respectively fixed at the upper frames 43, 44 and 45.

The structure of the upper frames 43, 44 and 45, and the connections between the wires and the upper frames may be changed variously.

Further, the frame 40 may have various structures not limited to the structure of FIG. 2.

The plate 50 is located at a center of the lower frame 41, and the subject 20 positions both feet on the plate 50 in an upright standing posture, and then equilibrium function of the subject 20 may be tested.

Here, the pressure measurement unit 100 may be disposed at the center of the plate 50 at which both feet of the subject 20 are positioned.

The pressure measurement unit 100 measures a plantar pressure of the subject and analyzes the plantar pressure. Here, the measured information may include a distribution of the plantar pressure, a size or magnitude of the plantar pressure, a mean value of the plantar pressure, a change of the plantar pressure and so on.

As illustrated in FIG. 1, the pressure measurement unit 100 includes a posture control part 110, a pressure measurement part 120, a pressure change analysis part 130 and a pressure information storage part 140.

The posture control part 110 adjusts the posture of the plate 50 to fit the posture of the subject and further initializes the pressure amendment unit 100, when the subject stands upright on the plate 50.

The posture control part 110 sets the posture of the plate 50 fitted to the posture of the subject to an initial posture, and initializes the pressure amendment unit 100 based on the initial posture.

Each subject has various standing postures due to various factors such as body shape and posture, and thus, the posture of the subject in a normal equilibrium state may vary for each subject. Thus, in the present example embodiment, considering the posture of the various normal equilibrium state of the subject, the posture of the plate 50 is adjusted. Based on the adjusted posture of the plate 50, the information of the pressure amendment unit 100 is initialized. Then, more accurate and suitable pressure information may be obtained for the subject.

In cases that the test is performed with the subject standing upright on the plate 50, the standing posture of the subject is changed according to the test performance. Thus, the pressure measurement part 120 measures the plantar pressure of the subject, according to the change of the stranding posture of the subject.

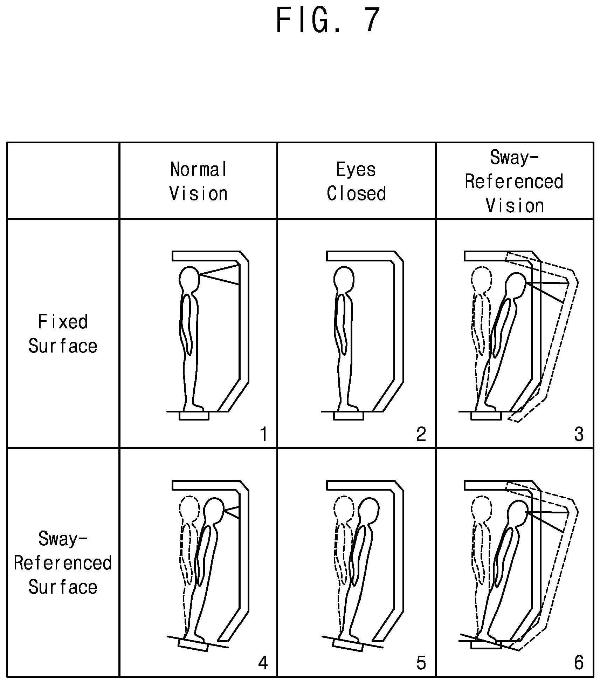

Equilibrium test on the 6 conditions as illustrated in FIG. 7 for deciding dizziness is performed with the subject standing upright on the plate 50. Here, the standing posture of the subject is changed as each condition, and then the pressure measurement part 120 measures the plantar pressure of the subject according to the change of the standing posture of the subject.

To measure the plantar pressure of the subject, the pressure measurement part 120 may include a pressure sensor. In addition, to measure the distribution of the plantar pressure, a plurality of pressure sensors may be disposed with a predetermined distance and arrangement in an area on which both feet are located.

The pressure change analysis part 130 analyzes the change of the pressure or the change of the pressure distribution, compared with the initial standing posture of the subject, when performing the equilibrium test for deciding dizziness, based on the plantar pressure or the distribution of the plantar pressure measured in the pressure measurement part 120.

In addition, the pressure or the distribution of the pressure measured in the pressure measurement part 120, and the pressure change or the change of the pressure distribution analyzed in the pressure change analysis part 130, are stored in the pressure information storage part 140.

The harness 60 is disposed in a predetermined space formed by the frame 40, and supports the body of the subject. In addition, the harness 60 is disposed over the plate 50.

Then, the equilibrium test for the subject is performed with the body of the subject, the upper portion of the subject, fixed by the harness 60. The harness 60 may be formed in a shape similar to a chair capable of fixing and supporting the subject's waist, upper body and so on, to support and fix the subject stably.

Here, the shape and the structure of the harness 60 may be changed variously.

In the present example embodiment, the balance measurement unit 200 is fixed at the harness 60, and the balance measurement unit 200 is explained below referring to FIG. 3.

Referring to FIG. 1 and FIG. 3, the harness 60 includes a pair of first and second fixing parts 61 and 62, and a third fixing part 63 connected to the first and second fixing parts 61 and 62. The first and second fixing parts 61 and 62 fixes the upper portion of the subject which is a pair of shoulders.

Here, the balance measurement unit 200 may be fixed at the third fixing part 63.

Alternatively, the balance measurement unit 200 may be fixed at the harness 60 and then the fixing state of the balance measurement unit 200 may be changed variously.

In the present example embodiment, the harness 60 fixes the upper portion of the subject, and at the same time, the balance measurement unit 200 measures a balance state which is the change of the posture as the test performance with the subject fixed at the harness 60.

In addition, in addition to the information on the pressure measured by the pressure measurement unit 100, the information on the balance state measured by the balance measurement unit 200 is used to evaluate or diagnose the posture balance of the subject, and thus the evaluation or the diagnosis on the posture balance of the subject may be performed more accurately.

The balance measurement unit 200 includes a balance information initialization part 210, a balance information measurement part 220, a balance change analysis part 230 and a balance information storage part 240.

The balance information initialization part 210 initializes the balance measurement unit 200 to fit the posture of the subject, when the subject stands upright on the plate 50 and the upper body of the subject is fixed at the harness 60.

The position or posture of the harness 60 is set to match the posture of the subject while being fixed to the subject, and thus the balance measurement unit 200 fixed at the harness 60 should be initialized based on the initial posture of the subject.

As explained above, each subject has a different standing posture depending on various factors such as body shape and posture, and thus a posture of the subject in a normal equilibrium state may be different for each subject. Thus, in the present example embodiment, the posture of the subject in various normal equilibrium states is considered, and then the information of the balance measurement unit 200 is initialized based on the equilibrium state of the harness 60 and the balance measurement unit 200 when the subject is fixed at the harness 60. Thus, more suitable and accurate information of the balance state for the subject may be obtained.

The balance information measurement part 220 measures the balance state of the subject according to the change of the standing posture of the subject or the change of the balance state of the subject, as the standing posture of the subject or the balance state of the subject is changed according to the test performance, with the subject fixed at the harness 60.

Here, the balance information measurement part 220, as illustrated in FIG. 3, may include at least one of accelerometer 201, gyroscope 202 and magnetometer 203.

In addition, the balance information measurement part 220 may further include a sensor control part 204 controlling the sensors, and a communication module 205 providing the measured information to the balance change analysis part 230 and the controller 300.

Then, the balance information measurement part 220 measures the balance state of the subject effectively using the sensors 201, 202 and 203, as the standing posture or the balance posture of the subject is changed according to the test performance.

As explained above, equilibrium test on the 6 conditions as illustrated in FIG. 7 for deciding dizziness is performed with the subject standing upright on the plate 50. Here, the standing posture or the balance state of the subject is changed as each condition, and then the balance information measurement part 220 measures the balance state of the subject according to the change of the standing posture or the balance state of the subject.

The balance change analysis part 230 analyzes the balance change in performing the equilibrium test for deciding dizziness, compared to the initial standing posture of the subject, based on the balance state information measured by the balance information measurement part 220.

In addition, the information on the balance state measured by the balance information measurement part 220 and the information on the balance change analyzed by the balance change analysis part 230 are stored by the balance information storage part 440.

Various kinds of information may be inputted to the information input part 70. For example, the information input part 70 may receive a subject identification information via identification means (not shown), a subject information by calling the stored subject information, information on height, weight, muscle mass, skeletal structure of the subject.

The inputted subject information may be considered in initializing the information of the subject posture or measuring the pressure or the balance state of the subject posture at the pressure measurement unit 100 or the balance measurement unit 200.

The controller 300 entirely controls the measurement of the pressure or the balance state at the pressure measurement unit 100 and the balance measurement unit 200. For example, the controller 300 may control operations of the posture control part 100, the pressure measurement part 120, the pressure change analysis part 130 and the pressure information storage part 140, or operations of the balance information initialization part 210, the balance information measurement part 220, the balance change analysis part 230 and the balance information storage part 240.

Further, the controller 300 may control the overall operation of the posture balance measurement system 10.

The evaluation part 400 evaluates and diagnoses the posture balance state of the subject, based on the results of the pressure change or the change of the pressure distribution of the subject obtained by the pressure measurement unit 100 and the results of the change of the balance state of the subject obtained by the balance measurement unit 200.

For 6 conditions used in the sensory organization test, as illustrated in FIG. 7, the evaluation part 400 evaluates and diagnoses the posture balance state or the dizziness of the subject, based on the pressure change or the change of the pressure distribution of the subject obtained by the pressure measurement unit 100 and he change of the balance state of the subject obtained by the balance measurement unit 200.

Further, although not shown in the figure, whether or not the subject has a disability, the subject's current condition, and furthermore, the result of the decision on the subject's equilibrium function, and the final judgment result or related information may be displayed on a display part (not shown).

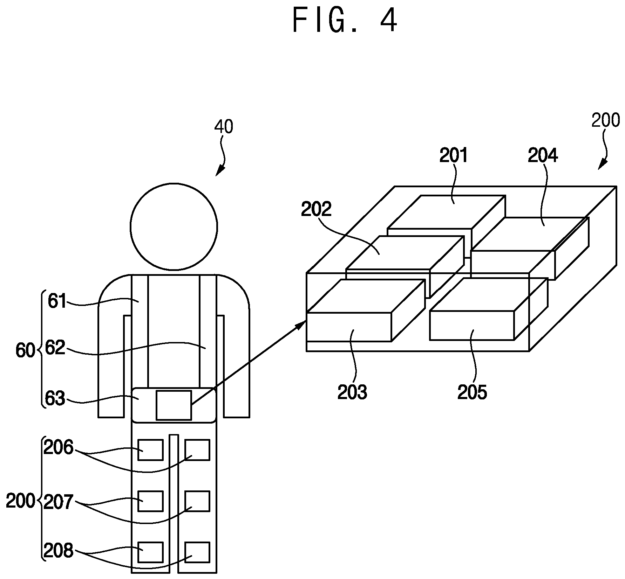

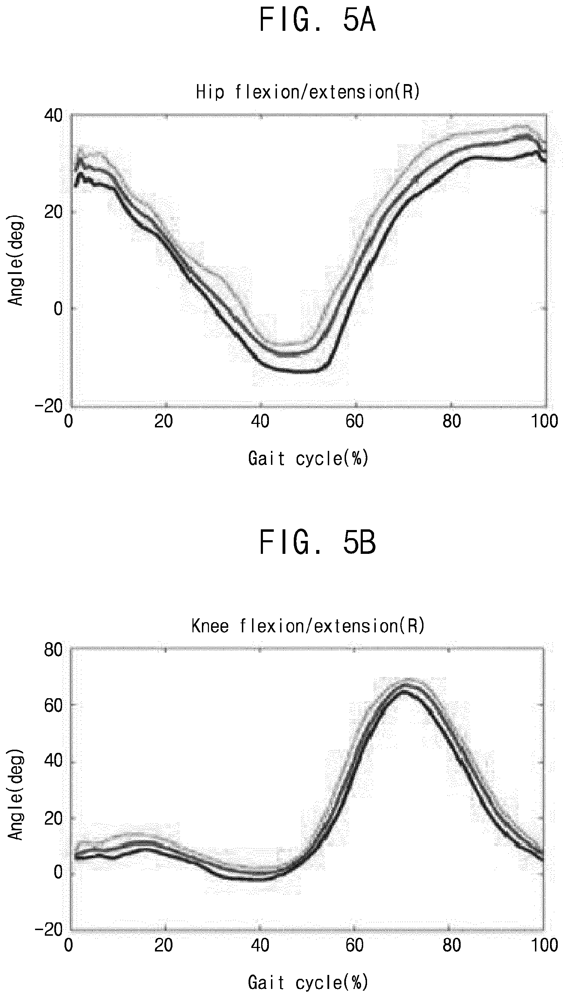

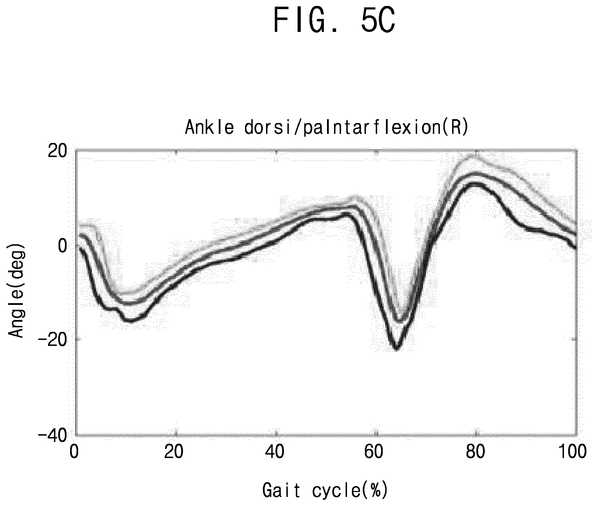

FIG. 4 is a schematic view illustrating another example state of the subject fixed to a harness and a hardware of a balance information measurement unit of FIG. 2. FIG. 5A, FIG. 5B and FIG. 5C are graphs showing example results of the posture balance measurement measured by the balance information measurement unit of FIG. 4.

Referring to FIG. 4, the balance information measurement part 200 may further include first to third joint sensors 206, 207 and 208, in addition to at least one of the accelerometer 201, gyroscope 202 and magnetometer 203 as illustrated in FIG. 3.

Here, the first joint sensor 206 may be disposed at a femoral region (thigh) of the subject, with a pair. The second joint sensor 207 may be disposed at a shin (shank) of the subject, with a pair. The third joint sensor 208 disposed at a foot of the subject, with a pair.

Each of the first, second and third joint sensors 206, 207 and 208 may be a inertial measurement device.

Thus, the first to third joint sensors 206, 207 and 208 sensors a joint angle between the leg joints of the subject 30. Here, the gyroscope 202 disposed at the waist of the subject 30 may sense the joint angle together with the first joint sensor 206.

The balance information measurement part 220 measures the balance state by measuring the joint angle of the subject using the first to third joint sensors 206, 207 and 208, in addition to the balance state of the subject 30 measured by the accelerometer 201, gyroscope 202 and magnetometer 203, as the standing posture or the balance posture of the subject is changed in the test performance.

Accordingly, the balance change analysis part 230 analyzes the change of the balance state or the change of the posture with the consideration of the joint angle of the subject simultaneously or separately in measuring the change of the balance state, in addition to the measurement of the change of the balance state of the subject based on the balance state measured by the balance information measurement part 220, as explained above.

FIG. 5A, FIG. 5B and FIG. 5C show examples of the change of the balance state or the change of the posture analyzed based on the information of the joint angle of the subject at the balance change analysis part 230.

FIG. 5A shows the example result of the joint angle between the lumbar (waist) and the thigh of the subject measured by the first joint sensor 206 together with the gyroscope 202. FIG. 5B shows the example result of the joint angle between the thigh and the shank of the subject measured by the first joint sensor 206 together with the second joint sensor 207. FIG. 5C shows the example result of the joint angle between the shank and the foot of the subject measured by the second joint sensor 207 together with the third joint sensor 208.

Further, the joint angle may be performed at each of both legs.

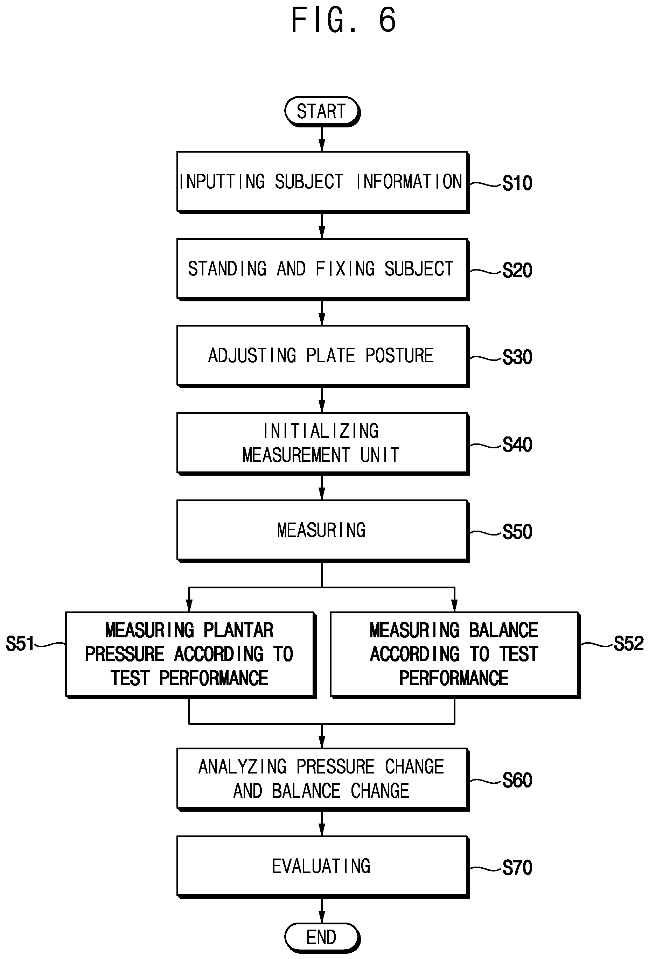

FIG. 6 is a flow chart showing a posture balance measurement method using the system of FIG. 1. FIG. 7 shows images of 6 conditions used in the sensory organization test.

Referring to FIG. 5, in the posture balance measurement method using the system 10 of FIG. 1, first, various kinds of information on the subject 30 are inputted to the information input part 70 (step S10).

Here, the inputted information may include various body information such as height, weight, muscle mass, skeletal structure and so on.

Then, the subject 30 stands upright on the plate 50, and the body of the subject 30 is fixed at the harness 60 (step S20).

Then, the posture control part 110 adjusts the posture of the plate 50 to fit the posture of the subject 30 (step S30). The posture control part 110 initializes the pressure measurement unit 100 based on the standing posture and the balance state of the subject 30, and the balance information initialization part 210 initializes the balance measurement unit 200 (step S40).

Then, the measurement is performed (step S50). Here, the pressure measurement part 120 measures the plantar pressure or the distribution of the plantar pressure of the subject according to the test performance (step S51), and the balance information measurement part 220 measures the balance information of the subject as the test performance (step S52).

Here, in the measuring (step S50), as illustrated in FIG. 7, the test is performed for 6 conditions used in the sensory organization test.

Then, the pressure change analysis part 130 analyzes the pressure change or the change of the pressure distribution based on the measured pressure or the measured pressure distribution (step S60), and the balance information analysis part 230 analyzes the balance change based on the measured balance information (step S60).

Then, the evaluation part 400 finally evaluates and diagnoses the posture balance of the subject, based on the analyzed results by the pressure change analysis part 130 and the balance information analysis part 230 (step S70).

According to the present example embodiments, conventional harnesses only serve to support the subject and plantar pressure is measured using only the lower plate, and thus when the equilibrium function is tested simply using only plantar pressure, the accuracy of the test may be decreased. Thus, the present example embodiments may solve the above problem.

In other words, by providing a balance measurement unit on a harness supporting the subject, it is possible to measure balance information as well as fixing the upper part of the subject, so that the postural balance of the subject may be more accurately measured together with the plantar pressure information.

Although the exemplary embodiments of the present invention have been described, it is understood that the present invention should not be limited to these exemplary embodiments but various changes and modifications can be made by one ordinary skilled in the art within the scope of the present invention as hereinafter claimed.

## Claims

1. A posture balance measurement system comprising:
a plate (50) configured to support a subject (30) in an upright standing posture;
a pressure measurement unit (100) configured to measure a plantar pressure of the subject (30);
a harness (60) configured to fix and support a body of the subject (30);
a balance measurement unit (200) disposed at the harness (60), and configured to measure balance information of the subject (30);
a controller (300) configured to control the pressure measurement unit (100) and the balance measurement unit (200) in accordance with the performance of a test; and
an evaluation part (400) configured to determine a posture balance state of the subject (30), based on pressure information of the subject (30) obtained by the pressure measurement unit (100), and the balance information of the subject obtained by the balance measurement unit (200),
wherein the balance measurement unit (200) comprises a balance information measurement part (220), and the balance information measurement part (220) is configured to measure a balance state of the subject (30) in which the subject's standing posture or balance posture changes according to the test performance when the subject (30) is fixed to the harness (60),
wherein the balance information measurement part (220) comprises a gyroscope (202) and at least one of an accelerometer (201) and a magnetometer (203) mounted on the harness (60), for measuring the balance state of the subject (30),
**characterised in that**:
1) the pressure measurement unit (100) is disposed on the plate (50), 2)
the balance information measurement part (220) further comprises a first joint sensor (206) disposed at a femoral region of the subject (30), a second joint sensor (207) disposed at a shin of the subject (30), and a third joint sensor (208) disposed at a foot of the subject (30),
3) the first joint sensor (206), together with the gyroscope (202), is configured to measure a joint angle between a waist and the femoral region of the subject (30),
4) the first joint sensor (206) is further configured to, together with the second joint sensor (207), measure a joint angle between the femoral region and the shin of the subject (30),
5) the second joint sensor (207) is further configured to, together with the third joint sensor (208), measure a joint angle between the shin and the foot of the subject (30).

2. The system of claim 1, wherein the pressure measurement unit (100) comprises:
a posture control part (110) configured to adjust a position of the plate (50) to fit a posture of the subject (30) and to initialize the pressure measurement unit (100), when the subject (30) stands upright on the plate;
a pressure measurement part (120) configured to measure the plantar pressure of the subject (30) according to the test performance;
a pressure change analysis (130) part configured to analyze a pressure change based on the measured plantar pressure; and
a pressure information storage part (140) configured to store the measured pressure and the pressure change.

3. The system of claim 1, wherein the balance measurement unit (200) comprises:
a balance information initialization part (210) configured to initialize the balance measurement unit (200) for the posture of the subject (30), when the subject stands upright on the plate;
a balance change analysis part (230) configured to analyze a balance change based on the measured balance state; and
a balance information storage part (240) configured to store the measured balance state and the balance change.

4. The system of claim 1, wherein the harness (60) comprises:
a pair of first and second fixing parts (61,62) configured to fix an upper portion of the subject (30); and
a third fixing part (63) connected to the first and second fixing parts (61,62), and configured to fix an abdomen portion of the subject (30).

5. The system of claim 4, wherein the balance measurement unit (200) is disposed at the third fixing part (63).

## Patentansprüche

1. Haltungs-Gleichgewichtsmesssystem, umfassend:
eine Platte (50), die so konfiguriert ist, dass sie ein Subjekt (30) in einer aufrecht stehenden Haltung stützt;
eine Druckmesseinheit (100), die so konfiguriert ist, dass sie einen Plantardruck des Subjekts (30) misst;
ein Gurtzeug (60), das so konfiguriert ist, dass es einen Körper des Subjekts (30) fixiert und stützt;
eine Gleichgewichtsmesseinheit (200), die an dem Gurtzeug (60) angeordnet und so konfiguriert ist, dass sie Gleichgewichtsinformationen des Subjekts (30) erfasst;
eine Steuerung (300), die so konfiguriert ist, dass sie die Druckmesseinheit (100) und die Gleichgewichtsmesseinheit (200) gemäß der Testausführung steuert; und
einen Auswertungsteil (400), der so konfiguriert ist, dass er einen Haltungs-Gleichgewichtszustand des Subjekts (30) basierend auf Druckinformationen des Subjekts (30), die von der Druckmesseinheit (100) erfasst werden, und die von der Gleichgewichtsmesseinheit (200) erfassten Gleichgewichtsinformationen des Subjekts bestimmt,
wobei die Gleichgewichtsmesseinheit (200) einen Gleichgewichtsinformationsmessabschnitt (220) umfasst, und der Gleichgewichtsinformationsmessabschnitt (220) so konfiguriert ist, dass er einen Gleichgewichtszustand des Subjekts (30) misst, bei dem sich die stehende Haltung oder Gleichgewichtshaltung des Subjekts gemäß der Testausführung verändert, wenn das Subjekt (30) an dem Gurtzeug (60) fixiert ist,
wobei der Gleichgewichtsinformationsmessabschnitt (220) ein Gyroskop (202) und mindestens eines aus einem Beschleunigungsmesser (201) und einem Magnetometer (203) umfasst, die an dem Gurtzeug (60) angebracht sind, um den Gleichgewichtszustand des Subjekts (30) zu messen,
**dadurch gekennzeichnet, dass**:
1) die Druckmesseinheit (100) an der Platte (50) angeordnet ist,
2) der Gleichgewichtsinformationsmessabschnitt (220) ferner einen ersten Gelenksensor (206), der im Bereich des Oberschenkels des Subjekts (30) angeordnet ist, einen zweiten Gelenksensor (207), der im Bereich des Unterschenkels des Subjekts (30) angeordnet ist, und einen dritten Gelenksensor (208), der im Bereich des Fußes des Subjekts (30) angeordnet ist, umfasst,
3) der erste Gelenksensor (206) zusammen mit dem Gyroskop (202) so konfiguriert ist, dass sie einen Gelenkwinkel zwischen der Taille und dem Oberschenkelbereich des Subjekts (30) messen,
4) der erste Gelenksensor (206) ferner so konfiguriert ist, dass er zusammen mit dem zweiten Gelenksensor (207) einen Gelenkwinkel zwischen dem Oberschenkelbereich und dem Schienbein des Subjekts (30) misst,
5) der zweite Gelenksensor (207) ferner, zusammen mit dem dritten Gelenksensor (208), so konfiguriert ist, dass sie einen Gelenkwinkel zwischen Schienbein und Fuß des Subjekts (30) messen.

2. System nach Anspruch 1, wobei die Druckmesseinheit (100) umfasst:
einen Haltungssteuerungsabschnitt (110), der so konfiguriert ist, dass er eine Position der Platte (50) an die Haltung des Subjekts (30) anpasst und die Druckmesseinheit (100) initialisiert, wenn das Subjekt (30) aufrecht auf der Platte steht;
einen Druckmessabschnitt (120), der so konfiguriert ist, dass er den Plantardruck des Subjekts (30) gemäß der Testausführung misst;
einen Druckänderungsanalyseabschnitt (130), der so konfiguriert ist, dass er eine Druckänderung basierend auf dem gemessenen Plantardruck analysiert; und
einen Druckinformationsspeicherungsabschnitt (140), der so konfiguriert ist, dass er den gemessenen Druck und die Druckänderung speichert.

3. System nach Anspruch 1, wobei die Gleichgewichtsmesseinheit (200) umfasst:
einen Gleichgewichtsinformationsinitialisierungsabschnitt (210), der so konfiguriert ist, dass er die Gleichgewichtsmesseinheit (200) für die Haltung des Subjekts (30) initialisiert, wenn das Subjekt aufrecht auf der Platte steht;
einen Gleichgewichtsänderungsanalyseabschnitt (230), der so konfiguriert ist, dass er eine Gleichgewichtsänderung basierend auf dem gemessenen Gleichgewichtszustand analysiert; und
einen Gleichgewichtsinformationsspeicherungsabschnitt (240), der so konfiguriert ist, dass er den gemessenen Gleichgewichtszustand und die Gleichgewichtsänderung speichert.

4. System nach Anspruch 1, wobei das Gurtzeug (60) umfasst:
ein Paar erster und zweiter Befestigungsteile (61, 62), die so konfiguriert sind, dass sie einen oberen Abschnitt des Subjekts (30) fixieren; und
einen dritten Befestigungsteil (63), der mit dem ersten und zweiten Befestigungsteil (61, 62) verbunden und so konfiguriert ist, dass er einen Abdomenabschnitt des Subjekts (30) fixiert.

5. System nach Anspruch 4, wobei die Gleichgewichtsmesseinheit (200) an dem dritten Befestigungsteil (63) angeordnet ist.

## Revendications

1. Système de mesure d'équilibre postural comprenant :
une plaque (50) conçue pour supporter un sujet (30) en posture debout ;
une unité de mesure de pression (100) conçue pour mesurer une pression plantaire du sujet (30) ;
un harnais (60) conçu pour fixer et supporter un corps du sujet (30) ;
une unité de mesure d'équilibre (200) disposée au niveau du harnais (60) et conçue pour mesurer les informations d'équilibre du sujet (30) ;
un dispositif de commande (300) conçu pour commander l'unité de mesure de pression (100) et l'unité de mesure d'équilibre (200) en fonction de la performance d'un test ; et
une partie d'évaluation (400) conçue pour déterminer un état d'équilibre postural du sujet (30), sur la base des informations de pression du sujet (30) obtenues par l'unité de mesure de pression (100) et des informations d'équilibre du sujet obtenues par l'unité de mesure d'équilibre (200),
dans lequel l'unité de mesure d'équilibre (200) comprend une partie de mesure d'informations d'équilibre (220) et la partie de mesure d'informations d'équilibre (220) est conçue pour mesurer un état d'équilibre du sujet (30) dans lequel la posture debout ou la posture d'équilibre du sujet change en fonction des performances du test lorsque le sujet (30) est fixé au harnais (60),
dans lequel la partie de mesure d'informations d'équilibre (220) comprend un gyroscope (202) et au moins un accéléromètre (201) et un magnétomètre (203) montés sur le harnais (60), servant à mesurer l'état d'équilibre du sujet (30),
**caractérisé en ce que** :
1) l'unité de mesure de pression (100) est disposée sur la plaque (50),
2) la partie de mesure d'informations d'équilibre (220) comprend en outre un premier capteur d'articulation (206) disposé au niveau d'une région fémorale du sujet (30), un deuxième capteur d'articulation (207) disposé au niveau d'un tibia du sujet (30) et un troisième capteur d'articulation (208) disposé au niveau d'un pied du sujet (30),
3) le premier capteur d'articulation (206), conjointement avec le gyroscope (202), est conçu pour mesurer un angle d'articulation entre une taille et la région fémorale du sujet (30),
4) le premier capteur d'articulation (206) est en outre conçu pour mesurer, conjointement avec le deuxième capteur d'articulation (207), un angle d'articulation entre la région fémorale et le tibia du sujet (30),
5) le deuxième capteur d'articulation (207) est en outre conçu pour mesurer, conjointement avec le troisième capteur d'articulation (208), un angle d'articulation entre le tibia et le pied du sujet (30).

2. Système selon la revendication 1, dans lequel l'unité de mesure de pression (100) comprend :
une partie de commande de posture (110) conçue pour ajuster une position de la plaque (50) pour s'adapter à une posture du sujet (30) et pour initialiser l'unité de mesure de pression (100), lorsque le sujet (30) se tient debout sur la plaque ;
une partie de mesure de pression (120) conçue pour mesurer la pression plantaire du sujet (30) en fonction de la performance du test ;
une partie d'analyse de changement de pression (130) conçue pour analyser un changement de pression sur la base de la pression plantaire mesurée ; et
une partie de stockage d'informations de pression (140) conçue pour mémoriser la pression mesurée et le changement de pression.

3. Système selon la revendication 1, dans lequel l'unité de mesure d'équilibre (200) comprend ;
une partie d'initialisation d'informations d'équilibre (210) conçue pour initialiser l'unité de mesure d'équilibre (200) servant à la posture du sujet (30), lorsque le sujet se tient debout sur la plaque ;
une partie d'analyse de changement d'équilibre (230) conçue pour analyser un changement d'équilibre sur la base de l'état d'équilibre mesuré ; et
une partie de mémorisation d'informations d'équilibre (240) conçue pour mémoriser l'état d'équilibre mesuré et le changement d'équilibre.

4. Système selon la revendication 1, dans lequel le harnais (60) comprend :
une paire de première et deuxième parties de fixation (61, 62) conçues pour fixer une partie supérieure du sujet (30) ; et
une troisième partie de fixation (63) reliée aux première et deuxième parties de fixation (61, 62) et conçue pour fixer une partie abdominale du sujet (30).

5. Système selon la revendication 4, dans lequel l'unité de mesure d'équilibre (200) est disposée au niveau de la troisième partie de fixation (63).
